# EUROPEAN PATENT APPLICATION

(11) **EP 0 769 292 A1**
(43) Date of publication of application: **23.04.1997**
(21) Application number: 96202724.9
(22) Date of filing: 30.09.1996
(51) Int. Cl.: A61K 7/48, A61K 7/06, A61K 35/78

(54) **Products for dermatologic care and cosmetics**

(30) Priority: 29.09.1995 EP 95202629
(71) Applicant: Sara Lee/DE N.V., 3532 AA Utrecht (NL)
(72) Inventor: Heinze, Friedrich, 2596 GG Den Haag (NL); Van Elk, Brigit Maria, 3061 HC Rotterdam (NL); Broekma, Adriaan, 2564 XP Den Haag (NL); Pronk, Johannes, 2725 AM Zoetermeer (NL); Deurloo, Catharina Jacoba Maria, 1071 HE Amsterdam (NL); Ligtvoet, Marijke Astrid, 2623 BK Delft (NL); Diepenhorst, Joke, 2273 XJ Voorburg (NL)
(74) Representative: Smulders, Theodorus A.H.J., Ir.

(57) **Abstract**

The present invention is directed to product for dermatologic or cosmetic care at least consisting of a matrix material comprising an amount of canola oil, a derivative of canola oil, a modification of canola oil or one or more components of canola oil.

## Description

The present invention is directed to dermatologic care and cosmetic products. More in particular this invention is directed to these products having improved properties with respect to their performance regarding protection of the skin against various damaging and/or harmfull influences.

Skin care products and cosmetic products can generally be divided into rinse-off products, such as cleansing products, and leave-on products, examples of which are decorative cosmetics, skin care and baby care products that exert their action for a longer period of time.

It is an object of the present invention to provide further improved, alternative skin care products, more in particular baby care products, especially having improved properties with respect to skin barrier function, anti-perspirant properties, decrease of skin roughness, moisturizing and/or regulation of skin penetration. It is a further object of the invention to provide a product of this type wherein at least part of the mineral oil, usually present in these products, has been replaced by naturally occurring products and/or derivatives of naturally occurring products.

The present invention is based on the surprising discovery that a specific combination of ingredients of naturally occurring products have a distinctly positive effect in dermatologic and skin care products, which combination has been found to occur in canola oil, canola oil formulations, complexes and/or matrices with canola oil. When used in skin care and cosmetic products canola oil accordingly provides at least one of the above mentioned properties to these products. It may even be that these products are even better than the products based on mineral oil.

The invention is accordingly directed to skin care products at least consisting of a matrix material comprising an amount of canola oil, a derivative of canola oil, a modification of canola oil or one or more components of canola oil or a canola oil/lipid/sterol solvent composition.

Canola oil is defined in JAOCS, vol. 61, no 6 June 1984), pp 1097-1101. This oil is characterised by a low level of erucic acid. The main fatty acid components in canola oil are oleic acid, linolic acid and linoleic acid. The amount of erucic acid components is below 5 % by weight. Most preferred is an amount of erucic acid residues of less than 1 % by weight. When the term 'canola oil' is used herein it is intended to encompass also derivatives, modifications and components thereof, as well as compositions, complexes or matrices with canola oil, derivatives, modifications and components thereof, unless otherwise indicated.

Derivatives of canola oil are i.a. the various reaction products of canola oil, for example the saponification products thereof or the reaction products thereof with other components, whereby the various properties and characteristics of canola oil are modified. Modification of canola oil encompasses more in particular the physical modification, such as purification (for example by crystalisation or extraction to yield a specific fraction) to increase the amount of unsaponifiable components, or to decrease the amount of erucic acid.

The matrix material containing canola oil as defined herein, also called skin lipid complex, may be canola oil, a derivative or modification thereof alone, or a combination with at least one other lipid matrix material, such as materials selected from triglycerides, glycerol, fatty acids, phytosterols, modified sterols, phospholipids and combinations thereof.

According to the invention canola oil can be used in a wide range of products. The presence of canola oil in these products generally exerts a positive effect on the skin, or compensates at least some of the negative effects of other ingredients, such as the negative effect of detergents, like sodium-lauryl-ether-sulfate(s).

Canola oil can also be used as a substitute for the mineral oil generally present in these types of products, which has among others the advantage that it is a fully natural product.

More in particular the use of canola oil, derivatives and chemical modifications thereof, has a positive effect in maintaining the natural functions of the skin, in keeping the skin in good condition for a long time and provides the products with caring and protecting properties for the skin.

The presence of canola oil in the products according to the invention provides an enhanced sin barrier function, a decrease in the skin roughness, as well as a regulated penetration, resulting among others in improved moisturizing, especially when combined with conventional moisturizing components. These effects, especially the barrier strengthening effects, are especially pronounced when compared to mineral oil based products. Without wishing to limit the invention thereto it is assumed that the astonishing occurrence of skin affinity, measurable in the form of high adhesiveness of the canola oil containing matrix, even after rinsing the skin with water, is responsible for the advantageous positive skin care/baby care effects.

Other effects of the use of canola oil are in strengthening skin, protecting skin, providing regulated barrier functions, decreasing transdermal waterloss and/or skin cleansing. The use of canola oil also provides anti-irritant and anti-inflammatory properties to the products of the present invention.

In case the skin care products contain components that have a negative influence on the skin, such as promoting roughness, dehydration, defatting and the like, the presence of canola oil may counteract this effect, or even compensate it more than fully, so it can provide anti-irritant cosmetic or dermatologic properties also. This may be the case with products containing an amount of synthetic detergent, such as shampoos or bath/shower products. Additionally the presence of the canola oil containing matrix may enhance the protective or healing properties of leave-on products.

According to the invention it is preferred to use canola oil as such, or a fraction thereof, in its natural unstabilised form. Canola oil as such is a natural product which is a distinct advantage over mineral oil. The use of the hydrogenated form of canola oil, although possible, is thus less preferred, although combinations thereof may offer advantages.

The canola oil can be used in a variety of products, for example caring and/or cleaning and/or decorative products, such as baby care products, moisturizing products, shampoo, skin conditioner, suncream, bath and/or shower cream or gel, soap bars, liquid soaps, hand lotion, nail-polish, nail-lacquer, lipstick, eye-shadow, body lotion, facial cream, talcum powder, foot powder, shaving cream, shaving soap, pre-shave, after-shave, salve, ointment, impregnated wipes and the like.

This means that the formulation basis can be selected from a large number of possibilities, depending on the intended use. Generally the term formulation basis is defined to encompass any material, not being canola oil, a fraction thereof or a derivative thereof, which is used in combination with the canola oil, a fraction thereof or a derivative thereof, as a support or attended material. This material may range from a woven or non-woven cloth or tissue, which can be impregnated with the oil, a lipophilic phase, or a dispersion such as a suspension or an emulsion. It is also possible to use foams, solutions, suspensions or gels, such as lipogels or hydrogels. The material may also be a powder, impregnated with the oil or a product containing the canola oil (such as baby powder or foot powder).

The products according to the present invention may be based on conventional ingredients, apart from the canola oil, whereby the actual composition will depend on the application. In general it is preferred that the canola oil in the matrix is used in admixture with other conventional skin care or cosmetic ingredients, whereby the matrix material itself may also be a suitable ingredient.

Examples of suitable ingredients are other lipids phospholipids, silicones, antioxidants soluble in the lipid phase like tocopherol and ascorbyl palmitate, lecithin, oil-soluble vitamins, such as Vitamin A, E or F. The composition may also contain other ingredients, such as emulsifiers, anti perspirant agents, stabilisers, preservatives, UV-filters, moisturizing agents, perfumes, colorants, softeners, waxes, pearling agents, mineral or organic fillers and other ingredients well known to the skilled cosmetic or dermatologic developer. In the formulations according to the invention, canola oil as defined herein, may be used as a replacement for at least part of the mineral oil conventionally used in the formulation.

The final formulation of products for cosmetic use or skin care, including dermatological use (cloth, powder, emulsion, wax, aerosol), depends on the specific application. As indicated, it is possible to impregnate a cloth or tissue with canola oil or a composition containing canola oil. The same applies for various powders used in skin care, such as talcum powder and foot powder.

It is also possible to use canola oil in semi-solid systems, such as waxes, or fluid systems, such as ointments, creams, gels, liquid-like lotions and milk, oily preparations and the like. In the case of ointments, which generally consist of a lipid phase alone or an emulsion, the canola oil constitutes, or will be present in the lipophilic phase. It is also possible to formulate liquid crystalline phases or liposomes/nanospheres with canola oil, with other skin relevant lipids, for example phospholipids, ceramides, polysilanes, squalanes, free fatty acids and/or sterols. Those sterols may be those present in the canola oil, but also others.

In the case of a water in oil emulsion the canola oil will be present in the lipid phase, which is the continuous phase. Combinations of canola oil and other lipids are generally preferred, although it is also possible to use canola oil in combination with one or more inorganic fillers, to provide the necessary viscosity.

It is to be noted that triple emulsions (oil-in-water-in-oil emulsions) are also included in the context of this invention, whereby the canola oil may be present in the outer and/or the inner lipid phase.

Fluid systems, such as bath or shower gels, shampoos and the like are frequently based on water as the continuous phase. In these products the canola oil, derivatives or modifications will preferably be present in dispersed (emulsified), solubilised or dissolved forms.

It is preferred to use canola oil in combination with at least one other component, as indicated above. Suitable systems include, in addition to the canola oil, glycerol, phytosterols and/or derivatives thereof, triglycerides and other lipids, such as squalane, squalene and isostearyl isostearate. An other system may be based on canola oil, partially hydrogenated canola oil, phytosterols, and other additives, such as antioxidants.

Preferred compositions of these systems are given in the following table in weight %, whereby column A designates a preferred range, column B a more preferred range and column C the most preferred range.

| | **A** | **B** | **C** |
|---|---|---|---|
| Glycerol | 0-90 | 0.5-80 | 5-70 |
| Phytosterols | 0-70 | 0-60 | 5-50 |
| Triglycerides | 0-50 | 0-40 | 1-25 |
| Other lipids | 0-70 | 0-50 | 0-25 |
| Others | 0-40 | 0.1-20 | 0.1-5 |
| Canola oil | 0.1-99.9 | 0.1-99 | 5-80 |

For the second system mentioned above the following compositions may be given:

| | **A** | **B** | **C** |
|---|---|---|---|
| Partially hydrogenated canola oil | 0-90 | 0.5-80 | 5-70 |
| Phytosterols | 0-70 | 0-50 | 0-25 |
| Others | 0-40 | 0.1-20 | 0.1-5 |
| Canola oil | 0.1-99.9 | 0.1-99 | 1-90 |

According to a preferred embodiment of the invention the products are baby care products, such as baby oil, baby powder, baby shampoo, baby creams, baby lotions, impregnated wipes, baby soap, baby ointment and the like. Canola oil has the property to repair damaged skin barrier function, which makes the use thereof most valuable in this area of care products. Thanks to the anti-irritant/anti-inflammatory properties the products provide a good protection against irritation by ammonia, other alkaline substances or skin incompatible materials.

The matrix material containing canola oil according to the invention also provides a good protection against physical or mechanical damage of the skin, such as caused by stripping of adhesive tape.

The use of a canola oil matrix material in the baby care products of the invention provides a ideal, natural protection, by building up and strengthening the natural skin barrier protection properties and the natural anti-irritant properties.

The various skin care products of the invention have all in common that they contain an amount of canola oil or a derivative thereof, preferably from 0.01 to 99 wt.% based on the total weight of the skin care product. A preferred upper limit is about 95 % by weight.

The products of the invention can be produced in a conventional manner. Especially in the case of the use of canola oil as replacement for mineral oil, one has to take into consideration the specific polarity of canola oil, for instance related to the viscosity of the final product. If necessary one may adjust the polarity of the system by using additional, less polar products such as various siloxanes. The long term temperature stability has also to be taken into consideration. Addition of specific antioxidants, well-known to the developer, could be advisable.

### Experimental work

The products of the present invention have been tested in relation to their influence on the Trans Epidermal Water Loss (TEWL).

Two different leave-on products (dermal lipid complex; Matrix A of the Examples and Matrix A with the canola oil replaced by mineral oil) were tested in a period of ten days, in which the skin of a volunteer's fore arm was tape stripped four times every morning. The tape stripped area and an untreated area were washed every morning and afternoon, followed by application of the leave-on products. Before and after the test the TEWL was determined, using an Evaporimeter EPI supplied by Servo Med.

Washing of the skin resulted in an increased TEWL, indicative of a loss of skin barrier function. Treatment of the skin with the dermal lipid complex with canola oil resulted in a significantly lower increase in TEWL than washed skin treated with the dermal lipid complex with mineral oil (p=0.03).

The same conclusion could be drawn for the test with stripping and washing (p=0.09). The results of the above tests are incorporated in the attached figure 1.

Pure canola oil and a leave-on product (dermal lipid complex; Matrix A of the Examples) were tested in a period of four days, in which the skin of a volunteer's fore arm was tape stripped seven times every morning. The tape stripped area was treated by applying the leave-on products twice a day. Before the test and the morning after the test period the TEWL was determined, using an Evaporimeter EPI supplied by Servo Med.

Stripping of the skin resulted in an increased TEWL. The treatment with pure canola oil resulted in a significantly (p<0.1) lower increase in TEWL. The treatment with the dermal lipid complex resulted in an even lower increase (p<0.1). In figure 2 the results of this test are given.
[X-axis: TEWL(stripped) - TEWL(untreated)]

The present invention is further elucidated on the basis of the following, non-limiting examples.

### EXAMPLE 1

| Matrix A | |
|---|---|
| Component | wt.% |
| Glycerol | 30 |
| PEG-10 Soy sterol | 15 |
| caprylic/capric triglycerides | 4 |
| Squalane | 10 |
| Phospholipid | 0.7 |
| Antioxidant | 0.3 |
| Canola oil | 40 |

### EXAMPLE 2

| Matrix B | |
|---|---|
| Component | wt.% |
| Phytosterol | 2 |
| Antioxidant | 0.3 |
| Phospholipid | 0.7 |
| Canola oil | 65 |
| Partially hydrogenated canola oil | 32 |

### EXAMPLE 3

| Powder blush | |
|---|---|
| Component | wt.% |
| Magnesium silicate | 15 |
| Kaolin | 5 |
| Canola Oil | 0.5 |
| Pigments | qs |
| Preservative and perfume | qs |
| Talcum | qs to 100 |

### EXAMPLE 4

| Baby oil | |
|---|---|
| Component | wt.% |
| Canola oil | 80 |
| Tocopherol | 0.5 |
| Perfume | qs |
| Mineral oil | qs to 100 |

### EXAMPLE 5

| Baby wipes - impregnation fluid | |
|---|---|
| Component | wt.% |
| PEG-40 Hydrogenated castor oil | 2.0 |
| Propylene glycol | 3.0 |
| Tocopherol acetate | 0.1 |
| Canola oil | 1.0 |
| Preservatives | qs |
| Perfume | qs |
| Water | qs to 100 |

### EXAMPLE 6

| Baby bodylotion | |
|---|---|
| Component | wt.% |
| Hexyldecanol | 0.5 |
| Hexyldecyl laurate | 1.0 |
| Glyceryl stearate | 5.0 |
| PEG-6-32 stearate | 3.0 |
| Dimethicone | 1.0 |
| Cetyl alcohol | 1.0 |
| Preservatives | qs |
| Perfume | qs |
| Matrix B | 10.0 |
| Water | qs to 100 |

### EXAMPLE 7

| Baby bath/shower gel | |
|---|---|
| Component | wt.% |
| Sodium laureth sulfate | 10.0 |
| Cocamidopropyl betaïne | 3.0 |
| Propylene glycol | 3.0 |
| Sodium chloride | 1.0 |
| Preservatives | qs |
| Perfume | qs |
| Matrix A | 10.0 |
| Water | qs to 100 |

### EXAMPLE 8

| Baby cream | |
|---|---|
| Cetearyl alcohol | 5.0 |
| Cetearylpolyglucose | 3.0 |
| Sodium stearyl lactylate | 1.0 |
| Glyceryl stearate | 2.0 |
| Stearic acid | 2.0 |
| Cetyl alcohol | 3.0 |
| Octyl stearate | 2.0 |
| Dicaprylyl ether | 1.0 |
| Dimethicone | 2.0 |
| Matrix A | 14.0 |
| Preservatives | qs |
| Perfume | qs |
| Water | qs to 100 |

### EXAMPLE 9

| Baby zinc ointment | |
|---|---|
| Canola oil | 10.0 |
| Zinc oxide | 8.0 |
| Petrolatum | 3.0 |
| Ozokerite | 0.7 |
| Hydrogenated castor oil | 1.0 |
| Glyceryl isostearate | 0.7 |
| Polyglyceryl-3 oleate | 0.2 |
| Dicocoyl pentaerythrityl distearyl citrate | 2.0 |
| Microcrystalline wax | 2.0 |
| Glyceryl oleate | 1.5 |
| Aluminium stearate | 0.3 |
| Propylene glycol | 4.0 |
| Dimethicone | 1.0 |
| Sorbitan sesquioleate | 2.5 |
| Magnesium sulfate | 0.5 |
| Matrix B | 5.0 |
| Water | qs to 100 |

### EXAMPLE 10

| Moisturising bodywash | |
|---|---|
| TEA lauryl sulfate | 10.0 |
| Cocamidopropyl betaïne | 3.0 |
| Glycerin | 5.0 |
| Sodium chloride | 1.5 |
| Matrix A | 2.0 |
| Preservative | qs |
| Perfume | qs |
| Water | qs to 100 |

### EXAMPLE 11

| Moisturising antibacterial bodyshampoo | |
|---|---|
| TEA lauryl sulfate | 10.0 |
| Cocamidopropyl betaïne | 3.0 |
| Triclosan | 0.3 |
| Glycerin | 5.0 |
| Sodium chloride | 1.5 |
| Matrix A | 1.5 |
| Preservative | qs |
| Perfume | qs |
| Water | qs to 100 |

### EXAMPLE 12

| Sunprotection cream | |
|---|---|
| C₁₂-C₁₅ alkyl benzoate | 10.0 |
| Isopropyl myristate | 3.0 |
| PPG-15 stearylether | 7.0 |
| Acrylate/C₁₀-C₃₀ alkylacrylatecrosspolymer | 0.3 |
| Carbomer | 0.3 |
| Octyl methoxycinnamate | 8.0 |
| Butyl methoxydibenzoylmethane | 2.0 |
| Triethanolamine | qs |
| Matrix A | 5.0 |
| Preservative | qs |
| Perfume | qs |
| Water | qs to 100 |

## Claims

1. Product for dermatologic or cosmetic care at least consisting of a matrix material comprising an amount of canola oil, a derivative of canola oil, a modification of canola oil or one or more components of canola oil.

2. Care product according to claim 1, wherein the matrix material also contains at least one other component selected from the group of lipid materials and derivatives thereof.

3. Care product according to claim 1 or 2, wherein the matrix material is combined with at least one active ingredient.

4. Care product according to claim 1-3, wherein the matrix material consist for 0.01 to 100 % by weight of canola oil, derivatives of canola oil, modifications of canola oil and/or one or more components of canola oil.

5. Care product according to claim 1-4, wherein canola oil, partially hydrogenated canola oil, hydrogenated canola oil or mixtures thereof are used.

6. Care product according to claim 1-5, wherein the matrix material and optional active ingredients have been applied to or incorporated in an inert support.

7. Care product according to claim 6, wherein the said inert support is selected from the group consisting of skin care acceptable solvents, water, pastes, powders woven or non-woven cloths, paper and combinations thereof.

8. Care product according to claim 1-7 in the from of a solution, an emulsion, an ointment, a cream, a spray, a stick, a powder, an impregnated cloth or an aerosol.

9. Care product according to claim 1-8, selected from the group consisting of baby care products, moisturizing products, shampoo, skin conditioner, suncream, bath and/or shower cream or gel, soap bars, liquid soaps, hand lotion, nail-polish, nail-lacquer, lipstick, eye-shadow, body lotion, baby powder, foot powder, shaving cream, shaving soap, pre-shave, after-shave and impregnated wipes.

10. Care product according to claim 2, wherein the said other component selected from the group of lipid materials and derivatives thereof, is selected from triglycerides, glycerol, fatty acids, phytosterols, modified sterols, phospholipids and combinations thereof.

11. Care product according to claim 1-10, wherein the product is selected from the group consisting of rinse-off products and leave-on products.

12. Care product according to claim 1-11, wherein the product is a baby care product.

13. Care product according to claim 1-12, wherein the amount of canola oil and/or derivative thereof ranges from 0.01 to 99 wt.% based on the total weight of the skin care product.

14. Use of an effective amount of canola oil, a derivative or a modification thereof in a skin care product for moisturizing skin.

15. Use of canola oil in baby care products.

16. Use of an effective amount of canola oil, a derivative or a modification thereof in a skin care product for regulation of penetration properties of the skin against irritating agents or skin incompatible components, strengthening skin, protecting skin, decreasing transdermal water-loss and/or skin cleansing.
